# EUROPEAN PATENT APPLICATION

(11) **EP 1 020 168 A1**
(43) Date of publication of application: **19.07.2000**
(21) Application number: 99100428.4
(22) Date of filing: 11.01.1999
(51) Int. Cl.: A61F 13/15

(54) **Disposable absorbent article with corrugated topsheet having filled corrugations**

(71) Applicant: THE PROCTER & GAMBLE COMPANY, Cincinnati, Ohio 45202 (US)
(72) Inventor: Giorgini, Gennaro, 64026 Roseto (IT)
(74) Representative: Hirsch, Uwe Thomas M.H.

(57) **Abstract**

The present invention relates to disposable absorbent articles such as sanitary napkins, pantiliners, baby diapers, adult incontinence articles, sweat pads and shoe inserts. According to the present invention the absorbent article on its wearer facing surface has a topsheet onto which liquid to be absorbed is provided during use of the article. The topsheet is provided with corrugations thereby forming troughs and peaks on the wearer facing surface of the topsheet corresponding to peaks and troughs respectively on the other surface of the topsheet. According to the present invention the troughs on the inside surface of the topsheet are at least partially filled with an absorbent material.

## Description

### Field of the invention

The present invention relates to disposable absorbent articles such as sanitary napkins, pantiliners, baby diapers, adult incontinence articles, sweat pads and shoe inserts. According to the present invention the absorbent article on its wearer facing surface has a topsheet onto which liquid to be absorbed is provided during use of the article. The topsheet is provided with corrugations thereby forming troughs and peaks on the wearer facing surface of the topsheet corresponding to peaks and troughs respectively on the other surface of the topsheet. According to the present invention the troughs on the inside surface of the topsheet are at least partially filled with an absorbent material.

### Background of the invention

Absorbent articles are well known in the art. Particularly sanitary napkins of all kinds of designs are well documented. Although the use of corrugations is well known in the art of disposable absorbent articles and has been disclosed previously such articles have not been successful or technically satisfactory as evidenced by the lack of commercially available embodiments.

Statutory invention declaration US - H - 1511 relates to absorbent articles and in particular sanitary napkins. The sanitary napkins contain a flow regulator positioned between the topsheet and the absorbent core in order to move liquid in the longitudinal direction to provide more effective use of the absorbent core. The flow regulator in this publication is provided by pleating or ring rolling a web in longitudinal direction. The napkin also provides softness, pliability and comfort in addition to the improved fluid distribution.

Case CM1036 discloses the use of a topsheet pleated in longitudinal direction in order to provide better liquid distribution, comfort and rewet of the absorbent structure. The pleats are also said to provide additional softness. The pleats in this publication have heights between 0.2 mm and 10 mm and examples of 3 mm and 5 mm are disclosed. PCT publication 96/00625 discloses various alternatives to provide a pleated substrate which is said to be useful as an external or internal layer of absorbent articles in order to improve liquid distribution, comfort and rewet. The alternatives provided include ring rolling, pleating and pressing in longitudinal direction.

Cases CM1099 and CM1100 disclose sanitary napkins or pantiliners which as a whole have been ring rolled in order to provide adaptability and extensibility of the product to the undergarment dimensions.

Based on these publications absorbent articles and in particular sanitary napkins are well know with pleated topsheets. The purpose of the pleating of such topsheets can be seen to be an improvement versus unpleated topsheets for softness, comfort, adaptability and pliability. However the hollow corrugations of the topsheet in such articles are subject to compression when considering the forces excerted onto such articles. This however diminishes their function in respect to their intended benefits.

Accordingly the present invention addresses the problem of further improving the comfort, pliability and softness of corrugated topsheets while at the same time providing an increased probability of providing such benefits to a wearer of such articles even under conditions in which the topsheet is compressed due to usual wearing of the article. In addition the total absorbent capacity of such absorbent articles can be improved when effectively filling hollow spaces with absorbent material. Accordingly the present invention improves the known corrugated topsheet articles in their original performance aspects and adds the aspect of durability, additional absorbency and improved efficiency of the absorbent structure according to the present invention.

### Summary of the invention

According to the present invention a disposable absorbent article such as a sanitary napkin having a wearer facing surface and a garment facing surface is provided which article comprises a topsheet, a backsheet joined to the topsheet and an absorbent core interposed between the topsheet and the backsheet. Each of the components topsheet, backsheet, absorbent core, have a wearer facing surface and a garment facing surface. The topsheet is folded or corrugated thereby creating troughs and peaks on the wearer facing surface of the topsheet and corresponding peaks and troughs respectively on the garment facing surface of the topsheet. According to the present invention the absorbent article has at least one of the troughs on the garment facing surface of the topsheet at least partially filled with an absorbent material. In an improved embodiment according to the present invention at least four troughs on the garment facing surface of the topsheet are at least partially filled with an absorbent material. In an even more preferred embodiment four troughs which are filled with absorbent material are disposed along the longitudinal side edges of the article thereby creating a barrier to liquid running of the side of the article.

In one aspect of the present invention the absorbent material filling the troughs on the garment facing surface of the topsheet increase the bending resistance of the article when comparing the same article without the absorbent material filling the troughs when bending perpendicular to the peaks. At the same time the bending resistance parallel to the peaks is not increased.

According to the present invention the absorbent material filling the troughs on the garment facing surface of the topsheet can be the same as the absorbent material of the absorbent core. Even preferable the absorbent core and the absorbent material in the troughs are formed integrally by providing the wearer facing surface of the absorbent core with troughs and peaks which are matched and aligned with the peaks and troughs on the garment facing surface of the topsheet. In another preferred embodiment according to the present invention the absorbent article has a longitudinal axis and perpendicular thereto a transverse axis and the troughs and peaks are substantially oriented along or parallel to the longitudinal axis of the article.

In accordance with one embodiment of the present invention the absorbent material can be expandable material which is expandable upon contact with liquid such as a regenerated compressed cellulose sponge. When using such a material for filling the troughs on the garment facing surface of the topsheet the troughs and peaks in the topsheet are collapsed but provide the necessary space to allow expanding of the absorbent material upon contact with liquid. In an alternative embodiment the absorbent material filling the troughs on the garment facing surface of the topsheet comprises absorbent gel material and most preferably absorbent gel material in fibrous form.

### Brief description of the drawing

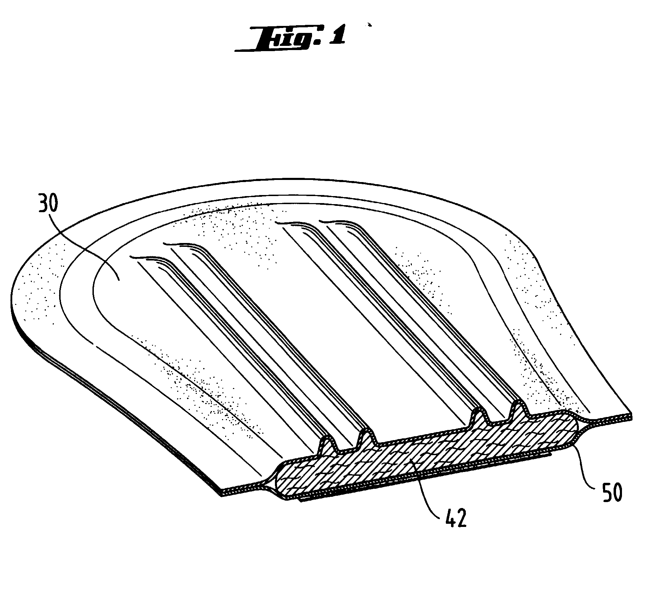

Figure 1 shows a perspective view of a sanitary napkin partially cut away in order to provide a cross sectional view of the internal parts of a sanitary napkin according to a preferred embodiment of the present invention.

### Detailed description of the invention

The present invention relates to absorbent disposable articles such as sanitary napkins, panty liners, incontinence products sweatpads and baby diapers. Typically such products comprise the elements of a liquid pervious topsheet, a backsheet and an absorbent core intermediate said topsheet and said backsheet. According to the present invention the topsheet, backsheet and core may be selected from any of the known types of these components provided that they meet the desired comfort and protection performance requirements and conditions noted below and in the apended claims.

In general, the topsheet should have good liquid retention to maintain a dry surface and thereby keep the skin of the wearer dry; the absorbent core needs to provide enough absorbent capacity and preferably allows the flow of vapour and/or air through it and the backsheet should prevent wet through (liquid impermeability) to retain the absorbed fluid while preferably being sufficiently breathable. Furthermore, the individual elements are joined, preferably using techniques such that the final product has the desired comfort and performance level.

In the following description of the invention the surface facing in the direction of the wearer is called wearer facing surface. In the drawing this direction is towards the top of the page. Further the surface facing in the direction of the garment is called garment lacing surface and in the drawing this direction is towards the bottom of the page.

### Absorbent article components

### The topsheet

According to the present invention the absorbent article comprises a folded or corrugated topsheet. The topsheets suitable for use herein comprises wovens, non-wovens, and three dimensional webs of a liquid impermeable polymeric film comprising liquid permeable apertures. In Figure1 the topsheet is indicated with reference numeral 30.

The topsheets for use herein may comprise a single layer or a multiplicity of layers. In a preferred embodiment the topsheet comprises a first layer which provides the user facing surface of the topsheet and a second layer between the first layer and the absorbent structure/core. In addition another layer on the wearer facing surface of the first layer but only extending in the central zone or in parts of the peripheral zone of the article can be desirable to provide extra softness or extra liquid handling/retaining abilities (this design is usually referred to as "hybrid topsheet"). The topsheet typically extends across the whole of the absorbent structure and can extend into and form part of or all of the preferred but optional sideflaps, side wrapping elements or wings. Also the topsheet (or rather at least one layer thereof) can wrap around the absorbent core, thereby providing a topsheet layer and a layer which is considered part of the backsheet.

The topsheet as a whole and hence each layer individually needs to be compliant, soft feeling, and non-irritating to the wearer's skin. It also can have elastic characteristics allowing it to be stretched in one or two directions. The topsheet has the principle function of acquisition and transport of fluid from the wearer towards the absorbent core and containment of the absorbent core. In addition to liquid permeability the topsheet should have a high vapour permeability and/or air permeability.

According to the present invention the layers of the topsheet can comprise wovens, non-wovens, but preferably a liquid permeable apertured polymeric film. One layer, but preferably the wearer facing and contacting layer, is provided by a film material having apertures which are provided to facilitate liquid transport from the wearer facing surface towards the absorbent structure. Such liquid permeable, apertured films are well-known in the art. They provide a resilient three dimensional fiber-like structure. Such films have been disclosed in detail for example in US 3 929 135, US 4 151 240, US 4 319 868, US 4 324 426, US 4 343 314, US 4 591 523, US 4 609 518, US 4 629 643, US 4 695 422 or WO 96/00548.

An example of such film is available from the Procter & Gamble Company, Cincinnati, Ohio, USA under the trade name Dryweave. Also such films are available from the Company Pantex from Pistoia, Italy under the designation "PF-films". Also film according to US 5 591 510 or WO 97/03118 and WO 97/03795 described for use as a layer in breathable backsheets can be employed but may require modification of the apertures to ensure liquid permeability from the wearer facing surface to the absorbent core which is the primary objective of the topsheet and the layers constituting it. Such modification can e.g. be a surface energy alteration which actively drives liquids into and through apertures by creating a gradient of surface tension of the film. A method to provide surface energy gradients is disclosed e.g. in WO 96/00548.

A particularly preferred design would then be to use the same film for the topsheet and the backsheet both possibly supplemented by additional layers. Such a film is e.g. wrapped around and encircles the absorbent core and is treated for liquid transport into the absorbent core in the area corresponding to the topsheet but is not treated in the area corresponding to the longitudinal sides and to the backsheet (or treated to prevent liquid migration from the absorbent core through the backsheet). Treatment in the area corresponding to the topsheet can e.g. provide a discontinuous coating of hydrophobic silicone on the wearer facing surface in line with WO 96/00548. In addition or alternatively the characteristics of the apertures can be made differently (i.e. for liquid transport) in the film area corresponding to the topsheet.

### Absorbent core

According to the present invention the absorbent cores suitable for use herein may be selected from any of the absorbent cores or core system known in the art. As used herein the term absorbent core refers to any material or multiple material layers whose primary function is to absorb, store and distribute fluid. In Figure 1 the absorbent structure is shown as a single layer 42.

The absorbent core of the present invention should have a high vapour permeability preferably also a high air permeability. The absorbent core preferably has a caliper or thickness of less than 12mm, preferably less than 8mm, more preferably less than 5mm, most preferably from 4mm to 2mm.

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components.

### Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs primarily not only in the thickness, but also along the length and width directions of the absorbent product. The primary distribution layer thus provides an isolation for liquids which have a posed through and improves the revert performance. The also optional but preferred secondary distribution layer typically underlies the primary distribution layer and is in fluid communication therewith. The purpose of this secondary distribution layer is to readily acquire fluid from the primary distribution layer and transfer it rapidly to all regions of the underlying storage layer. This helps the fluid capacity of the underlying storage layer to be fully utilised. The fluid distribution layers can be comprised of any material typical for such distribution layers.

### b Fluid Storage Layer

Positioned in fluid communication with, and typically underlying the primary or secondary distribution layers, is a fluid storage layer (42). The fluid storage layer can comprise any usual absorbent material or combinations thereof It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise particles of a substantially water-insoluble, slightly cross-linked, partially neutralised, polymeric gelling material. This material forms a hydrogel upon contact with water Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate. These layers can be joined to each other for example by adhesive or melting a polymeric powder binder (e.g. PE powder), by mechanical interlocking, or by hydrogen bridge bonds. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, fibers with surface capillaries, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer.

Non-homogeneous distribution can also refer to laminates of carriers enclosing absorbent gelling materials partially or fully.

An alternative are foam like or actual foam structures as liquid storage. There are open cell foams which absorb liquid and through chemical or surface interaction retain the liquid also under pressure. Typical foams in this context are e. g. those disclosed in PCT publications WO 93/03699, WO 93/04092, WO 93/04113. Another alternative which in particularly preferred in the context of the present invention are compressed sponge material such as those disclosed in EP-A-804.917. Such foam and sponge materials can be used without the optional primary/secondary distribution layers and can extend into the folds or corrugations of the topsheet as shown in Figure 1.

### c Optional Fibrous ("Dusting") Layer

An optional component for inclusion in the absorbent core according to the present invention is a fibrous layer adjacent to, and typically underlying the storage layer identified by reference numeral 42 in Figure 1. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent core according to the invention, and preferably is provided close to or as part of the primary or secondary fluid distribution layer or the fluid storage layer, are odor control agents such as zeolites, carbon black, silicates, EDTA or other chelates. Such agents are preferably provided in particulate form or as part of particles and can be provided together with the absorbent gelling material mentioned supra.

### Backsheet

The absorbent article according to the present invention also comprises a backsheet (50). The backsheet primarily has to prevent the extrudes absorbed and contained in the absorbent structure from wetting articles that contact the absorbent product such as underpants, pants, pyjamas, undergarments, and shirts or jockets, thereby acting as a barrier to fluid transport. In addition however, the backsheet according to the preferred embodiment of the present invention permits the transfer of at least water vapour, preferably both water vapour and air through it and thus allows the circulation of air into and water vapour out of the article. The backsheet typically extends across the whole of the absorbent structure and can extend into and form part or all of sideflaps, side wrapping elements or wings, if present.

For the preferred embodiment according to the present invention suitable breathable backsheets for use herein comprise at least one impervious polymeric backsheet layer. The backsheet comprises a resilient three dimensional web which consists of a liquid impermeable film which has apertures and is air permeable. Preferred breathable backsheets for use herein are those having a high vapour exchange, most preferably both a high vapour and high air exchange. The film is oriented such that it retards or prevents liquid from passing from the absorbent core towards the outside while allowing free air flow through it.

In such an embodiment any additional backsheet layer needs to provide at least water vapour permeability so as to support breathability of the article. It is not required but desirable that it also supports air permeability in order to further improve the comfort benefit from the breathability of the article. In this context suitable water vapour and air permeable layers include two-dimensional micro- or macro-apertured films, which can also be micro-or macroscopically expended films, formed apertured films and monolithic films, as well as nonwovens, or wovens. Such films are disclosed in detail e.g. in EPO 293 482 and the references therein, or US 3, 929,135, US 4 637 819 and US 4 591 523. Preferably this film layer is made in accordance with the aforementioned US-A-5,591,510 or PCT WO- 97/03818, WO-97/03795. In particular, this layer comprises a polymeric film indicated in figure 1 as layer (50), having capillaries. The preferred capillaries extend away from the wearer facing surface of film (50) at an angle which is less then 90 degrees.

### Filled topsheet corrugations

According to the present invention the topsheet is corrugated or folded thereby providing a number of spaces on the inside of the absorbent article. Preferably but not necessarily the corrugations or folds are parallel, and of equal dimensions to each other. This would provide repetitive spaces on the inside of the absorbent article. These spaces inside the absorbent article correspond to peaks on the outside of the topsheet of the article and are filled with an absorbent material. This absorbent material can be any of the materials listed under the description of the absorbent core element or combinations thereof.

The primary benefit of the filling of the peaks of the topsheet from the inside of the absorbent article is that the corrugation is stabilized and thereby can provide a better wiping effect when moved perpendicular to the corrugation direction across the skin. Even small movements and shifts, for example when walking with a sanitary napkin installed in the undergarment, will thereby provide a wiping benefit which takes off liquid or not yet absorbed moisture, e.g. sweat, from the skin. If the filling of the peaks of the topsheet from the inside of the absorbent article is provided with an absorbent material this does provide the benefit that the peak being in contact and moving across the skin will draw liquid into the article. If then this filling material is able to transport liquid towards the absorbent fluid storage layer (as is desirable) it will be available for further acquisition situations.

Separately the corrugation increases the topsheet surface and by filling the peaks of the topsheet from the inside with absorbent material the complete additional topsheet surface area is able to acquire liquid and thereby increase drastically the speed of acquisition. Simultaneously any liquid which is squeezed out of the article through the topsheet ( so called rewet) is more likely to be squeezed out in the troughs of the topsheet thereby being in a location where no direct contact with the skin exists and where additional acquiring surface (with absorbent material behind it) is available on the sides of the corrugations. And finally filling the peaks of the topsheet from the inside of the absorbent article does of course provide additional absorbent capacity generally allowing for better performance in absorbing liquids by the article.

In a preferred embodiment according to the present invention the filling material is integral with the absorbent core. This can be achieved by making it integral with one of the optional primary or secondary fluid distribution layers or by making it integral with the fluid storage layer according to the above description of the absorbent core element.

As shown in the drawing the absorbent core simply extends into the topsheet corrugations. In a even more preferred embodiment the corrugations are provided primarily on the sides of the absorbent article such as shown in figure 1. By providing these filled corrugations on the sides the additional benefit of reducing the probability of liquid running off the side edges of the article during usual usage can be reduced.

The corrugations do not have to be absolutely parallel and could also be provided in cross direction on the article. This may be more appropriate when considering articles such as under arm sweat pads but is a possibility within the present invention.

When filling the peaks of the topsheet from the inside of the absorbent article by a compressed sponge material, which is a preferred embodiment according to the present invention, care should be taken to provide enough expansion space (or little enough expanding material) to allow full utilization of the expansion capability of the absorbent material.

### Absorbent article construction

According to the present invention at least two, preferably all of the elements of the topsheet, backsheet and absorbent core elements article are joined to each other. The elements or layers are joined together across their common interface. In this manner the topsheet is joined to the absorbent core, and the core is joined to the backsheet. Furthermore, each of said topsheet, backsheet and core elements may comprise more than one layer and these layers may also be similarly joined. In addition the topsheet may be directly or indirectly joined to the backsheet at the periphery of the absorbent article to contain the absorbent core.

The elements and layers thereof may be joined by any means known in the art for affixing two adjacent layers of material, such that the layers are directly attached to one another or directly attached to one another via the joining means. Suitable joining means include adhesive, fusion bonding, ultra sonic bonding, stitching, heat (e.g. thermobonding by welding fibers at intersections or melting a polymer to attach fibers or films to each other), embossing, crimping, pressure bonds, dynamic mechanical bonds or combinations thereof.

Especially if the absorbent article finds utility as a sanitary napkin or panty liner, the absorbent article is also provided with a panty fastening means which provides means to attach the article to an undergarment. For example the panty fastening means may comprise a mechanical fastener such as hook and loop fasteners such as marketed under the tradename VELCRO, snaps or holders. Alternatively, the article is fastened to the undergarment by means of panty fastening adhesive on the backsheet. The panty fastening adhesive provides a means for securing the article to the panty and preferably a means for securing the article when soiled, to a fold and wrap package for convenient disposal.

According to the present invention the absorbent article can be used beneficially in the context of sanitary napkins, panty liners, incontinence articles, sweatpads and diapers. However, sanitary napkins and pantyliners are particularly susceptible to the present invention. The disposable article may thus also have all those features and parts which are typical for products in the context of their intended use. For sanitary napkins this includes particularly wings or side flaps which are provided on the side edges of the napkin and which fold around the crotch edge of an undergarment. The side flaps can be provided as extensions of one or several of the elements of the napkin such as the topsheet and backsheet. They can also be made separately and be joined to the side margin of the napkin.

## Claims

1. Disposable absorbent article having a wearer facing surface and a garment facing surface and comprising
a topsheet having
a wearer facing surface and a garment facing surface,
a backsheet having a wearer facing surface and a garment facing surface and being joined to said topsheet and,
an absorbent core having a wearer facing surface and a garment facing surface and being interposed between said topsheet and said backsheet,
said topsheet being in a folded or corrugated configuration creating troughs and peaks on said wearer facing surface of said topsheet corresponding to peaks and troughs respectively on said garment facing surface of said topsheet and said absorbent article being characterised in that
at least one of said troughs on said garment facing surface of said topsheet is at least partially filled with an absorbent material.

2. Disposable absorbent article according to any of the preceding claims wherein there are at least 4 troughs on said garment facing surface of said topsheet which are at least partially filled with said absorbent material.

3. Disposable absorbent article according to any of the preceding claims wherein said absorbent material also provides increased resistance against bending of said article perpendicular to said peaks and troughs relative to the same article without said absorbent material filling said troughs on said garment facing surface of said topsheet while the resistance against bending parallel to said peaks and troughs is not increased relative to the same article without said absorbent material filling said troughs on said garment facing surface of said topsheet.

4. Disposable absorbent article according to any of the preceding claims wherein said absorbent core comprises the same material as said absorbent material, preferably said absorbent core and said absorbent material are formed integrally by providing said wearer facing surface of said absorbent core with troughs and peaks which are matched and aligned with said peaks and troughs on said garment facing surface of said topsheet such that said peaks of said absorbent core provide said absorbent material filling said troughs on said garment facing surface of said topsheet.

5. Disposable absorbent article according to any of the preceding claims wherein said article has a longitudinal axis and perpendicular thereto a transverse axis, and said troughs and peaks are substantially, parallel to said longitudinal axis.

6. Disposable absorbent article according to any of the preceding claims wherein said troughs and peaks are collapsed and said absorbent material is expandable upon contact with liquid, preferably said absorbent material is a regenerated, compressed cellulose sponge.

7. Disposable absorbent article according to any of the preceding claims wherein said absorbent material comprises absorbent gel material, preferably in fibrous form.

8. Disposable absorbent article according to any of the preceding claims wherein said article is a sanitary napkin.
